(19) Europäisches Patentamt
European Patent Office
Office européen des brevets

(11) Veröffentlichungsnummer: **0 047 949**
**B1**

(12) # EUROPÄISCHE PATENTSCHRIFT

(45) Veröffentlichungstag der Patentschrift: **27.07.83**

(51) Int. Cl.³: **C 07 C 69/63,** C 07 C 69/708, C 07 C 69/36, C 07 C 67/313

(21) Anmeldenummer: **81107001.0**

(22) Anmeldetag: **07.09.81**

(54) **Verfahren zur Herstellung perfluorierter Carbonsäurefluoride.**

(30) Priorität: **12.09.80 DE 3034549**

(43) Veröffentlichungstag der Anmeldung: **24.03.82 Patentblatt 82/12**

(45) Bekanntmachung des Hinweises auf die Patenterteilung: **27.07.83 Patentblatt 83/30**

(84) Benannte Vertragsstaaten: **BE CH DE FR GB IT LI NL**

(56) Entgegenhaltungen:
**DE-B-1 274 581**
**CHEMICAL ABSTRACTS, Band 80, Nr. 17, 29. April 1974, Seite 350, Nr. 95151p Columbus, Ohio, U.S.A. I.L. KNUNYANTS et al.: »Perfluoropropylene oxide in reactions with electrophilic reagents«**
**INORGANIC CHEMISTRY, Band 3, Nr. 2, 27. Januar 1964, Seiten 287–288, Washington, U.S.A. M. LUSTIG et al.: »Preparation of an acyl fluoride from an O-fluorosulfate; some new O-fluorosulfates«**

(73) Patentinhaber: **HOECHST AKTIENGESELLSCHAFT, Postfach 80 03 20, D-6230 Frankfurt/Main 80 (DE)**

(72) Erfinder: **Schwertfeger, Werner, Dr., Erlenstrasse 8, D-6306 Langgöns (DE)**

**EP 0 047 949 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Verfahren zur Herstellung perfluorierter Carbonsäurefluoride

Perfluorierte Carbonsäurefluoride sind hauptsächlich Zwischenprodukte in der organischen Fluorchemie. Wenn in ihnen die Struktureinheit

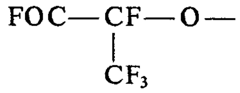

vorliegt, können sie etwa durch Pyrolyse direkt in perfluorierte Vinylverbindungen überführt werden:

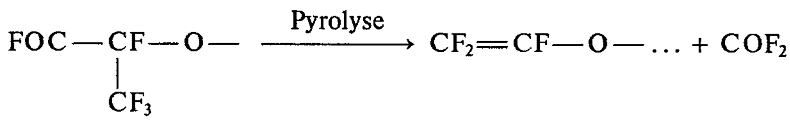


Wenn die besagte Struktureinheit nicht vorliegt, kann sie z. B. durch Umsetzung des perfluorierten Carbonsäurefluorids mit Hexafluorpropenepoxid auf bekannte Weise erzeugt werden:

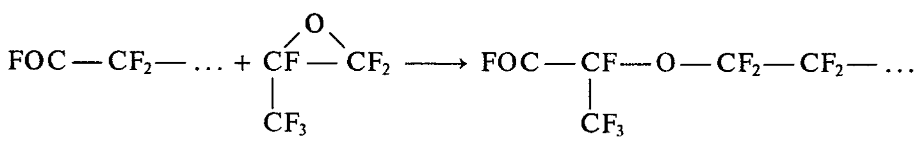

Die perfluorierten organischen Vinylverbindungen sind durch Homo- und Copolymerisation in wertvolle, chemisch und thermisch außerordentlich beständige Oligo- und Polymere überführbar. Die Oligomeren mit flüssiger Konsistenz dienen z. B. als Schmier- und Gleitmittel sowie Hydraulikflüssigkeiten, die Polymeren mit fester Konsistenz u. a. als Überzugsmaterialien, Elastomere, Ionenaustauscher (wenn noch basische oder saure Gruppen vorhanden sind) etc., und zwar überall dort, wo besonderer Wert auf chemische und thermische Resistenz gelegt wird.

Perfluorierte Carbonsäurefluoride mit noch einer weiteren funktionellen Gruppe im Molekül sind z. B. perfluorierte Dicarbonsäurefluoridester. Die Estergruppe kann dann auf übliche Weise etwa in die freie Säuregruppe überführt werden, so daß sich perfluorierte Dicarbonsäurefluoridester insbesondere als Ausgangs- bzw. Zwischenprodukte für die Herstellung von perfluorierten Ionenaustauschern eignen.

Zahlreiche perfluorierte Dicarbonsäurefluoridester sowie Verfahren zu deren Herstellung sind bereits bekannt. Etwa nach dem in der DE-OS 2 651 531 beschriebenen Verfahren werden solche perfluorierten Dicarbonsäurefluoridester durch Umsetzung von Perfluorlactonen mit einem Alkohol erhalten:

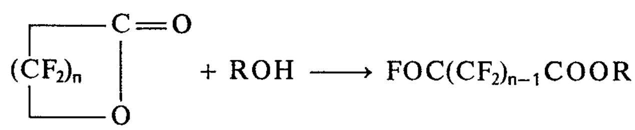

R = Alkyl
n = ganze Zahl von 2–4

Die Anwendbarkeit dieses Verfahrens ist jedoch dadurch sehr eingeschränkt, daß technisch nur das Perfluorbutyrolacton (n = 3) zugänglich ist, und zwar entweder aus $J(CF_2)_3COF$ (DE-AS 2 642 824) oder aus $J(CF_2)_4J$ (DE-AS 2 635 312), in letzterem Falle nur im Gemisch mit großen Mengen an Perfluortetrahydrofuran und mit Perfluorbernsteinsäuredifluorid, das von dem Lacton destillativ kaum abtrennbar ist.

Das Verfahren der DE-OS 2 708 677 geht aus von perfluorierten Dicarbonsäurefluoridestern der Formel:

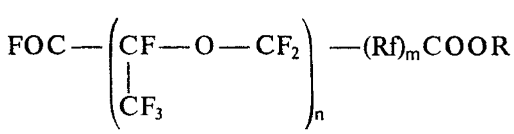

worin

R = Alkyl
Rf = bifunktionelle ($C_1 - C_{10}$)-Perfluorgruppe
m = 0 oder 1
n = 1–5;

daraus werden durch Umsetzung mit einem Alkalicarbonat die entsprechenden Carboxylat-Ester und aus diesen durch Pyrolyse dann perfluorierte Vinylverbindungen hergestellt:

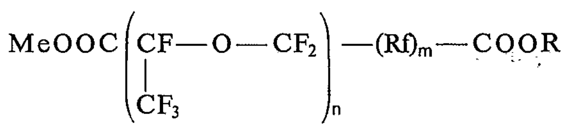

(Me = Alkalimetall)

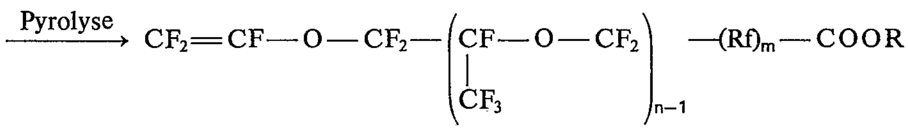


Perfluorierte Dicarbonsäurefluoridester der Formel:

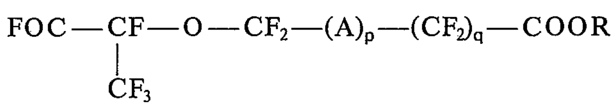

worin

A = bifunktionelle ($C_1 - C_{10}$)-Perfluorgruppe
R = organ. Gruppen
p = 0 oder 1
q = 1–8

sind bekannt aus der DE-OS 2 751 050; sie werden hergestellt durch Umsetzung der entsprechenden perfluorierten Dicarbonsäure-Difluoride mit Alkohol.

Dieses Verfahren läuft jedoch nicht selektiv zu den perfluorierten Dicarbonsäurefluoridestern ab, sondern führt stets zu Gemischen mit den schwer abtrennbaren, isomeren Halbestern und den Diestern, die zusammen bis zu etwa 30% ausmachen können und die für die Herstellung der perfluorierten Vinylverbindungen nicht brauchbar sind.

Die DE-OS 2 817 366 beschreibt perfluorierte Dicarbonsäurefluoridester der folgenden Formel:

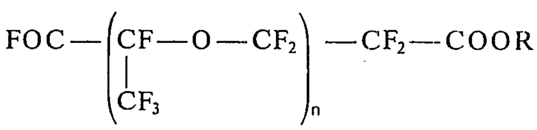

worin R = ($C_1$–$C_6$)-Alkyl und
n = 0–6.

Die Verbindungen werden wie folgt hergestellt:

mit n = 0:

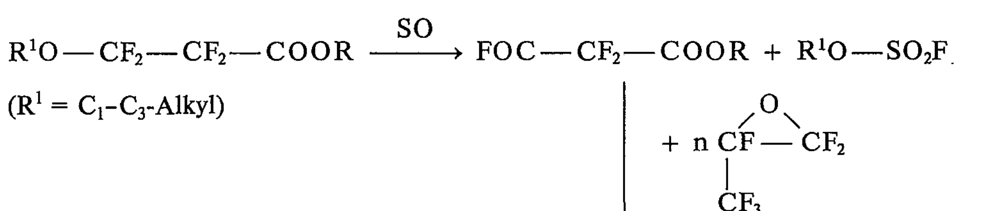


mit n = 1–6:

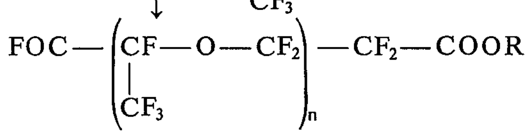

Praktische Bedeutung besitzt dieses Verfahren nur mit der Ausgangsverbindung

$$CH_3O-CF_2-CF_2-COOCH_3.$$

Das bei der Umsetzung mit $SO_3$ daraus in stöchiometrischer Menge entstehende Nebenprodukt $CH_3OSO_2F$ ist eine hochtoxische Verbindung, deren Gefährlichkeit mit Dimethylsulfat und ähnlichen Methylierungsmitteln vergleichbar ist [Alder, R. W. et al., Chem. Eng. News 56, 37, 56 (1978)].

Die Säurefluoridgruppe kann in perfluorierten organischen Verbindungen auch durch Zersetzung der Fluorsulfatogruppe in Gegenwart von Alkalifluoriden erzeugt werden.

In Inorg. Chem. 3 287 (1964) ist die Zersetzung eines primären Fluorosulfats mit KF beschrieben.

$$CF_3-\underset{\underset{OSO_2F}{|}}{CF}-CF_2-OSO_2F \xrightarrow[60°C,\ 16\ h]{KF} CF_3-\underset{\underset{OSO_2F}{|}}{CF}-COF + SO_2F_2$$

Mengenangaben fehlen. Alle weiteren dort beschriebenen Beispiele führen zur vollständigen Zerstörung der Ausgangsverbindung, oder das eingesetzte Fluorosulfat wird vollständig zurückerhalten.

In Inorg. Chem. 4 1441 (1965) werden primäre Fluorosulfate mit einem etwa 50molaren Überschuß an KF pro Mol Fluorosulfat umgesetzt.

$$NF_2-CF_2-CF_2-OSO_2F \xrightarrow[RT,\ 18\ h]{KF} NF_2-CF_2-COF + SO_2F_2$$

$$CF_3-\underset{\underset{NF_2}{|}}{CF}-CF_2-OSO_2F \xrightarrow[RT,\ 15\ h]{KF} CF_3-\underset{\underset{NF_2}{|}}{CF}-COF + SO_2F_2$$

J. Fluorine Chem. 14, 519 (1979) beschreibt die Umsetzung von primären Fluorosulfaten mit Caesiumfluorid.

$$Rf-OCF_2-CF_2-OSO_2F \xrightarrow[14\ h,\ 110°C]{CsF} Rf-O-CF_2-COF + SO_2F_2$$

$$Rf = CF_3,\ SF_5$$

Bei dieser Reaktion werden das Fluorosulfat und das CsF im Molverhältnis von 1 : 20 eingesetzt.

Allen diesen Reaktionen ist gemeinsam, daß ohne Lösungsmittel gearbeitet wird. In diesen Fällen scheint ein erheblicher Überschuß an Alkalifluorid für das Gelingen der Reaktion erforderlich zu sein.

In Izv. Akad. Nauk SSSR, Ser. Khim. 1973 2659 (engl. Ausgabe) wurde gezeigt, daß für solche Reaktionen auch katalytische Mengen an Alkalifluorid verwendet werden können, wenn ein aprotisches polares Lösungsmittel wie Diglyme oder Acetonitril eingesetzt wird:

$$CF_3-CO-CF_2OSO_2F \xrightarrow[\text{Lösungsmittel}]{KF} CF_3-CO-COF + SO_2F_2$$

Durch eigene Versuche wurde festgestellt, daß die Reaktion von $\omega$-Fluorsulfatoperfluorcarbonsäureestern mit katalytischen Mengen an Alkalifluorid in Gegenwart eines Lösungsmittels wie Tetraglyme nicht zu den erwarteten Perfluordicarbonsäurefluoridestern führt, sondern neben vollständiger Zersetzung des Ausgangsproduktes Perfluordicarbonsäuredifluorid und Perfluordicarbonsäurediester liefert (siehe Vergleichsbeispiel).

Es war aufgrund dieses Befundes und der oben angeführten Literaturbeispiele für die Spaltung von primären Fluorosulfaten nun überraschend, daß $\omega$-Fluorsulfatoperfluorcarbonsäureester in Gegenwart von katalytischen Mengen Alkalifluorid und in Abwesenheit eines aprotischen polaren Lösungsmittels mit hoher Ausbeute in Perfluordicarbonsäurefluoridester überführt werden können.

Erfindungsgegenstand ist somit ein Verfahren zur Herstellung perfluorierter Carbonsäurefluoride durch Zersetzung perfluorierter Fluorsulfatoverbindungen in Gegenwart mindestens eines Alkalifluorids, das dadurch gekennzeichnet ist, daß man als perfluorierte Fluorsulfatoverbindungen Verbindungen der Formel I

$$FSO_2-O(CF_2)_m-\left(CF_2-O-\underset{\underset{CF_3}{|}}{CF}\right)_n-COOR \qquad (I)$$

worin

R = Alkyl, Aryl, Aralkyl mit vorzugsweise bis zu 10 C-Atomen, insbes. $CH_3$ oder $C_2H_5$,
m = ganze Zahl von 1 – 10, vorz. 1 – 8, insbes. 1 – 6, und
n = 0 oder ganze Zahl von 1 – 10, vorz. 0 – 3, insbes. 0 oder 1,

verwendet sowie daß man die Zersetzung in Gegenwart nur katalytischer Alkalifluorid-Mengen und in Abwesenheit von aprotischen polaren Lösungsmitteln durchführt.

Aus den Verbindungen I entstehen so in hohen Ausbeuten die perfluorierten Carbonsäurefluoride mit noch einer Estergruppe im Molekül der Formel II:

$$FOC-(CF_2)_{m-1}-\left(CF_2-O-\underset{\displaystyle CF_3}{\underset{|}{CF}}\right)_n-COOR \qquad \text{(II)}$$

worin R, m und n die gleiche Bedeutung wie in Formel I besitzen.

Die Ausgangsverbindungen I für das erfindungsgemäße Verfahren werden mit besonderem Vorteil nach dem Verfahren der gleichzeitig eingereichten Patentanmeldung EP-A-47 950 dadurch hergestellt, daß man

a) $\omega$-H-Perfluorcarbonsäurehalogenide der Formel III:

$$H(CF_2)_m-\left(CF_2-O-\underset{\displaystyle CF_3}{\underset{|}{CF}}\right)_n-COA' \qquad \text{(III)}$$

worin m und n die gleiche Bedeutung wie in den Formeln I und II besitzen und A' = Halogen in einem aus einem Gemisch aus Fluorsulfonsäure $FSO_3H$ und einem von deren Alkalisalzen bestehenden Elektrolyten unter Verwendung von Metallen der Platingruppe (Osmium, Iridium, Platin) und/oder von glasartigem Kohlenstoff als Anodenmaterial und von einem üblichen, jedoch unter den Elektrolysebedingungen stabilen Kathodenmaterial elektrolysiert, die dabei gebildeten $\omega$-Fluorsulfato-perfluorcarbonsäurehalogenide der Formel IV

$$FSO_2-O-(CF_2)_m-\left(CF_2-O-\underset{\displaystyle CF_3}{\underset{|}{CF}}\right)_n-COA' \qquad \text{(IV)}$$

worin m und n wiederum die gleiche Bedeutung wie in den Formeln I und II und A' die gleiche Bedeutung wie in Formel III besitzen, isoliert und

b) mit einer organischen Hydroxylverbindung der Formel V

ROH (V)

worin R die bei Formel I genannte Bedeutung besitzt, zu $\omega$-Fluorsulfato-perfluorcarbonsäureestern der Formel I verestert.

Katalysatoren für das erfindungsgemäße Verfahren sind die Alkalifluoride (LiF, NaF, KF, RbF, CsF).

Die Katalysatoren können sowohl einzeln als auch in Mischung miteinander eingesetzt werden. Die Katalysatormenge liegt im allgemeinen zwischen etwa 1 und 50 Mol-%, vorzugsweise zwischen etwa 10 und 30 Mol-%, bezogen auf die Ausgangsverbindung I.

Die Reaktion wird ohne jedes Lösungsmittel durchgeführt.

Die Reaktionstemperaturen liegen je nach dem verwendeten Katalysator im allgemeinen in dem Bereich zwischen etwa $-20$ und $+120°$ C.

Es kann sowohl bei Normaldruck als auch unter Überdruck gearbeitet werden.

Für die erfindungsgemäße Reaktion ist es praktisch ohne Belang, in welcher Reihenfolge die Reaktionskomponenten zusammengegeben werden. Es ist allerdings von Vorteil, während der gesamten Dauer der Reaktion durch gutes Rühren für eine gute Durchmischung des Ansatzes zu sorgen.

Nach einer bevorzugten Ausführungsart gibt man den Katalysator und das Fluorosulfat I zusammen und erhitzt langsam, bis Gasentwicklung auftritt. Nach beendeter Gasentwicklung wird der Ansatz über eine Kolonne destilliert.

Die nach dem erfindungsgemäßen Verfahren hergestellten perfluorierten Carbonsäurefluoride II

mit noch einer Estergruppe im Molekül sind im allgemeinen farblose, hydrolyseempfindliche Flüssigkeiten. Daher ist die Herstellung unter Ausschluß von Feuchtigkeit durchzuführen.

Wegen der hohen, meist über etwa 90% d. Th. liegenden Ausbeuten und der hohen Reinheit der Endprodukte II stellt die Erfindung einen erheblichen Fortschritt auf diesem Gebiet dar.

Die Verbindungen II werden nach bekannten Methoden hauptsächlich zu perfluorierten Vinylverbindungen mit noch einer Estergruppe im Molekül verarbeitet, die ihrerseits zu wertvollen Homo- und Copolymerisaten umgesetzt werden. Die Homo- und Copolymerisate werden u. a. (nach Verseifung der Estergruppen) als chemisch und thermisch widerstandsfähige Ionenaustauscher verwendet.

Die Erfindung wird nun durch die folgenden Beispiele näher erläutert. Nach den Erfindungsbeispielen (A) folgt noch ein Vergleichsbeispiel (B), welches zeigt, daß die erfindungsgemäße Reaktion in Gegenwart von Lösungsmittel nicht mehr funktioniert.

## Erfindungsbeispiele

### Beispiel 1

Methoxalylfluorid

$CH_3OOC-COF$

Die Reaktion wird in einem Abzug durchgeführt.

In einem trockenen Kolben mit Magnetrührer, Thermometer, Rückflußkühler und Blasenzähler werden bei Raumtemperatur 5,8 g (0,1 Mol) trockenes, fein pulverisiertes Kaliumfluorid und 104 g (0,5 Mol) Fluorsulfatodifluoressigsäuremethylester vorgelegt. Der Ansatz wird unter gutem Rühren erhitzt. Ab 50°C Innentemperatur beginnt Sulfurylfluorid zu entweichen. Nachdem die Gasentwicklung beendet ist, wird noch eine Stunde unter Rückfluß erhitzt. Die anschließende Destillation über eine Füllkörperkolonne liefert 48 g (90,5%) Methoxalylfluorid mit Kp 91°C (760 torr).

Analyse: Ber. C 33,98 H 2,85 F 17,91
Gef. C 34,00 H 2,80 F 17,80

$^1H$-NMR ($CDCl_3$): 4.00 (s)
$^{19}F$-NMR ($CDCl_3$)*): +23.8 (−COF)
IR (neat): 5.35 μ (COF), 5.64 μ (COO)

*) Bei allen $^{19}F$-NMR-Spektren dient $CFCl_3$ als innerer Standard.

### Beispiel 2

Carbomethoxydifluoressigsäurefluorid

$CH_3OOC-CF_2-COF$

Die Reaktion wird in einem Abzug durchgeführt.

In einem trockenen Kolben mit Magnetrührer, Thermometer, Rückflußkühler und Blasenzähler legt man bei Raumtemperatur 5,8 g (0,1 Mol) trockenes, fein pulverisierbares Kaliumfluorid und 128 g (0,496 Mol) ω-Fluorsulfatoperfluorpropansäureethylester vor. Beim anschließenden Erhitzen tritt ab 55°C Innentemperatur Gasentwicklung ($SO_2F_2$) auf. Nachdem die Gasentwicklung beendet ist, wird noch eine Stunde unter Rückfluß erhitzt. Während der gesamten Reaktion wird der Ansatz gut gerührt. Die Destillation liefert 70 g (90,4%) Carbomethoxydifluoressigsäurefluorid mit Kp 86°C (760 torr).

Analyse: Ber. C 30,79 H 1,94 F 36,52
Gef. C 31,05 H 1,95 F 35,60

$^1H$-NMR ($CDCl_3$): 4.00 (s)
$^{19}F$-NMR ($CDCl_3$): +22.6 (COF), −110.9 ($CF_2$)
IR (Gasspektrum): 5.3 μ (COF), 5.52 μ (−COO−)

## Beispiel 3

### Carboethoxydifluoressigsäurefluorid

$C_2H_5OOC-CF_2-COF$

Die Reaktion wird wie für Carbomethoxydifluoressigsäurefluorid beschrieben durchgeführt.

Es werden 5,8 g (0,1 Mol) Kaliumfluorid und 100 g (0,35 Mol) 3-Fluorsulfatoperfluorpropansäuremethylester eingesetzt. Die Destillation liefert 57 g (95,8%) Carboethoxydifluoressigsäurefluorid mit Kp 98° C (760 torr).

| | | | | |
|---|---|---|---|---|
| Analyse: | Ber. | C 35,31 | H 2,96 | F 33,51 |
| | Gef. | C 35,20 | H 3,00 | F 33,10 |

| | |
|---|---|
| $^1$H-NMR ($CDCl_3$): | 1.40 (t, 3 H, J = 7 Hz), 4.45 (q, 2 H, J = 7 Hz) |
| $^{19}$F-NMR ($CDCl_3$): | +22.6 (COF), −111 ($CF_2$) |
| IR (neat): | 5.32 (COF), 5.59 (−COO−) |

## Beispiel 4

### 4-Carbomethoxyperfluorbutansäurefluorid

$CH_3OOC-(CF_2)_3-COF$

Die Reaktion wird in einem Abzug durchgeführt.

In einem trockenen Kolben mit Magnetrührer, Thermometer, Rückflußkühler und Blasenzähler gibt man 78 g (0,21 Mol) 5-Fluorsulfatoperfluorpentansäuremethylester und 3,0 g (0,053 Mol) Kaliumfluorid. Der Ansatz wird langsam erhitzt. Ab ca. 55° C Innentemperatur wird Sulfurylfluorid frei. Nach dem Ende der Gasentwicklung ersetzt man den Rückflußkühler durch eine Füllkörperkolonne mit Kolonnenkopf. Mit Kp 117° C (768 torr) erhält man bei der Destillation 49,5 g (88,4%) 4-Carbomethoxyperfluorbutansäurefluorid.

| | | | | |
|---|---|---|---|---|
| Analyse: | Ber. | C 28,14 | H 1,18 | F 51,93 |
| | Gef. | C 28,25 | H 1,15 | F 51,95 |

| | |
|---|---|
| $^1$H-NMR ($CDCl_3$): | 3.97 (s) |
| $^{19}$F-NMR ($CDCl_3$): | +25.44 (1F, COF), −117.9 (4F, $CF_2$), −123 · 6 (2F, $CF_2$) |
| IR (neat): | 5.33 μ (−COF), 5.69 μ (−COO−) |

## Beispiel 5

### 6-Carbomethoxyperfluorhexansäurefluorid

$CH_3OOC-CF_2-CF_2-CF_2-CF_2-CF_2-COF$

Die Reaktion wird in einem Abzug durchgeführt.

In einen trockenen Kolben, der mit Magnetrührer, Thermometer, Vigreux-Kolonne, Kolonnenkopf und nachgeschalteter Kältefalle (−78° C) versehen ist, gibt man 25 g (0,055 Mol) 7-Fluorsulfatoperfluorheptansäuremethylester und 0,6 g (0,01 Mol) Kaliumfluorid. Der Ansatz wird langsam erhitzt. Ab ca. 60° C Innentemperatur tritt Gasentwicklung auf. Nachdem die Gasentwicklung beendet ist, wird zur Destillation angeheizt. Mit Kp 150−151° C (746 mm) werden 15,6 g (80%) 6-Carbomethoxyperfluorhexansäurefluorid erhalten.

| | | | | |
|---|---|---|---|---|
| Analyse: | Ber. | C 26,98 | H 0,85 | F 58,69 |
| | Gef. | C 26,7 | H 0,8 | F 57,9 |

| | |
|---|---|
| $^1$H-NMR ($CDCl_3$): | 3.89 (s) |
| $^{19}$F-NMR ($CDCl_3$): | +25.2 (1F, COF), −118.6 (4F, $-CF_2-COOCH_3$ und $-CF_2-CO-$), −121.6 (2F, $CF_2$), −123.0 (4F, 2X$CF_2$) |
| IR (neat): | 5.30 μ (−COF), 5.59 μ (−COO−) |

Beispiel 6

7-Carbomethoxy-6-oxaperfluoroctansäurefluorid

$$FOC-CF_2-CF_2-CF_2-CF_2-O-\underset{\displaystyle CF_3}{\overset{|}{CF}}-COOCH_3$$

Die Reaktion wird in einem Abzug durchgeführt.

In einem trockenen Kolben mit Magnetrührer, Thermometer, Rückflußkühler und Blasenzähler werden 96 g (0,18 Mol) 8-Fluorsulfatoperfluor-2-methyl-3-oxooctansäuremethylester und 5,2 g (0,09 Mol) fein pulverisiertes, trockenes Kaliumfluorid vorgelegt. Beim Erwärmen des Gemisches tritt ab ca. 60° C Innentemperatur Gasentwicklung auf. Nachdem die Gasentwicklung beendet ist, wird noch 1 Stunde auf 80–90° C erhitzt. Die anschließende Destillation über eine Füllkörper-Kolonne liefert 71 g (93%) Säurefluorid mit Kp 50° C (12 torr).

Analyse: Ber. C 25,16 H 0,72 F 58,51
Gef. C 25,50 H 0,70 F 58,80

$^1$H-NMR ($CDCl_3$): 3.95 (s)
$^{19}$F-NMR ($CDCl_3$): +27.5 (COF), −78.1 (1F, $-O-CF_2-$, Jgem = 148 Hz), −81.6 ($CF_3$), −85.5 (1F, $-O-CF_2-$, Jgem = 148 Hz), −118.3 ($-CF_2-CO-$), −122.8 ($CF_2$), −124.9 ($CF_2$), −130.2 (CF)
IR (neat): 5.32 μ (COF), 5.59 μ (−COO−)

Vergleichsbeispiel

Die Reaktion wird in einem Abzug durchgeführt.

In einem trockenen Kolben mit Magnetrührer, Thermometer, Tropftrichter, Vigreux-Kolonne, Kolonnenkopf und Blasenzähler legt man bei Raumtemperatur 7,5 g (0,05 Mol) Caesiumfluorid und 50 ml Tetraglyme vor. Dazu tropft man 56 g (0,156 Mol) 5-Fluorsulfatoperfluorbutansäuremethylester. Es setzt sofort Gasentwicklung ein. Der Ansatz erwärmt sich. Anschließend wird zur Destillation angeheizt. Auch dabei ist eine leichte Gasentwicklung zu beobachten. Das IR-Spektrum des abgehenden Gases zeigt, daß unter anderem $CO_2$, $SO_2F_2$ und Säurefluorid(e) vorhanden sind. Die Destillation liefert 8,5 g Flüssigkeit mit Kp 40–50° C, die nach dem IR-Spektrum aus verunreinigten Perfluorpentandicarbonsäuredifluorid bestehen. Es wird kein 4-Carbomethoxyperfluorbutansäurefluorid erhalten. Im Rückstand wird Perfluorpentandicarbonsäuredimethylester IR-spektroskopisch nachgewiesen.

**Patentansprüche**

1. Verfahren zur Herstellung perfluorierter Carbonsäurefluoride der Formel II

$$FOC-(CF_2)_{m-1}-\left(CF_2-O-\underset{\displaystyle CF_3}{\overset{|}{CF}}\right)_n-COOR \qquad (II)$$

worin

R = Alkyl, Aryl, Aralkyl und vorzugsweise bis zu 10 C-Atomen, insbesondere $CH_3$ oder $C_2H_5$,
m = ganze Zahl von 1–10, vorzugsweise 1–8, insbesondere 1–6, und
n = 0 oder ganze Zahl von 1–10, vorzugsweise 0–3, insbesondere 0 oder 1,

durch Zersetzung perfluorierter Fluorsulfatoverbindungen in Gegenwart mindestens eines Alkalifluorids, dadurch gekennzeichnet, daß man als perfluorierte Fluorsulfatoverbindungen Verbindungen der Formel I

$$FSO_2-O-(CF_2)_m-\left(CF_2-O-\underset{\displaystyle CF_3}{\overset{|}{CF}}\right)_n-COOR \qquad (I)$$

worin R, m und n die gleiche Bedeutung wie in Formel II besitzen, verwendet, sowie daß man die

Zersetzung in Gegenwart nur katalytischer Alkalifluorid-Mengen und in Abwesenheit von Lösungsmitteln durchführt.

2. Verfahren nach Anspruch 1, dadurch gekennzeichnet, daß die Alkalifluorid-Mengen 1—50 Mol-%, vorzugsweise 10—30 Mol-%, bezogen auf die Ausgangsverbindung I, betragen.

**Claims**

1. A process for the preparation of perfluorinated carboxylic acid fluorides of the formula II

$$FOC-(CF_2)_{m-1}-\left(CF_2-O-\underset{\displaystyle CF_3}{\underset{|}{CF}}\right)_n-COOR \qquad (II)$$

in which R = alkyl, aryl or aralkyl, with preferably up to 10 C-atoms, in particular $CH_3$ or $C_2H_5$, m = integer from 1—10, preferably 1—8, in particular 1—6, and n = 0 or an integer from 1—10, preferably 0—3, in particular 0 or 1, by decomposition of perfluorinated fluorosulfato compounds in the presence of at least one alkali metal fluoride, characterized in using as the perfluorinated fluorosulfato compounds compounds of the formula I

$$FSO_2-O-(CF_2)_m-\left(CF_2-O-\underset{\displaystyle CF_3}{\underset{|}{CF}}\right)_n-COOR \qquad (I)$$

in which R, m and n have the same meaning as in formula II and carrying out the decomposition in the presence of only catalytic amounts of alkali metal fluoride(s) and in the absence of solvents.

2. A process as claimed in claim 1, characterized in the amounts of alkali metal fluoride(s) being 1—50 mole %, preferably 10—30 mole %, relative to the starting compound I.

**Revendications**

1. Procédé de préparation de fluorures d'acides carboxyliques perfluorés de formule II:

$$FOC-(CF_2)_{m-1}-\left(CF_2-O-\underset{\displaystyle CF_3}{\underset{|}{CF}}\right)_n-COOR \qquad (II)$$

(dans laquelle R est un groupe alkyle, aryle, aralkyle ayant de préférence jusqu'à 10 atomes de carbone, notamment $CH_3$ ou $C_2H_5$;

m est un nombre entier valant 1 à 10, avantageusement 1 à 8, notamment 1 à 6, et
n est nul ou est un nombre entier valant 1 à 10, avantageusement 0 à 3, notamment 0 ou 1),

par décomposition de composés fluorosulfatoperfluorés en présence d'au moins un fluorure alcalin, procédé caractérisé en ce qu'on utilise, comme composés fluorosulfatoperfluorés, des composés de formule I:

$$FSO_2-O-(CF_2)_m-\left(CF_2-O-\underset{\displaystyle CF_3}{\underset{|}{CF}}\right)_n-COOR \qquad (I)$$

(dans laquelle R, m et n ont le même sens que pour la formule II), et en ce qu'on effectue la décomposition en présence de quantités catalytiques seulement d'un fluorure alcalin et en l'absence de solvants.

2. Procédé selon la revendication 1, caractérisé en ce que les quantités de fluorures alcalins sont de 1 à 50 moles %, avantageusement 10 à 30 moles %, par rapport au composé I de départ.